# EUROPEAN PATENT APPLICATION

(11) **EP 0 809 990 A1**
(43) Date of publication of application: **03.12.1997**
(21) Application number: 97600003.4
(22) Date of filing: 31.03.1997
(51) Int. Cl.: A61F 6/04

(54) **Protective sheath**

(30) Priority: 02.04.1996 GR 96010113
(71) Applicant: Delibassis, Christos, 111 44 Athens (GR)
(72) Inventor: Delibassis, Christos, 111 44 Athens (GR)

(57) **Abstract**

Protective sheath for oral sex, which comprises an elastic sheath (1) with shaping accepting tongue and lips and held in front of the mouth with a plastic wire-like frame (2) which is kept in position by an elastic cord (3) round the head (Fig. 1, 2, 3, 4) or self-adhesive ribbons (4) [Fig. 1, 2, 3, 4] Also by a self- adhesive ribbon (5) round the rim (Fig. A1, C1).

This sheath provides protection of love-makers from the passage from the one to the other of AIDS, Hepatitis and Germs during oral sex.

## Description

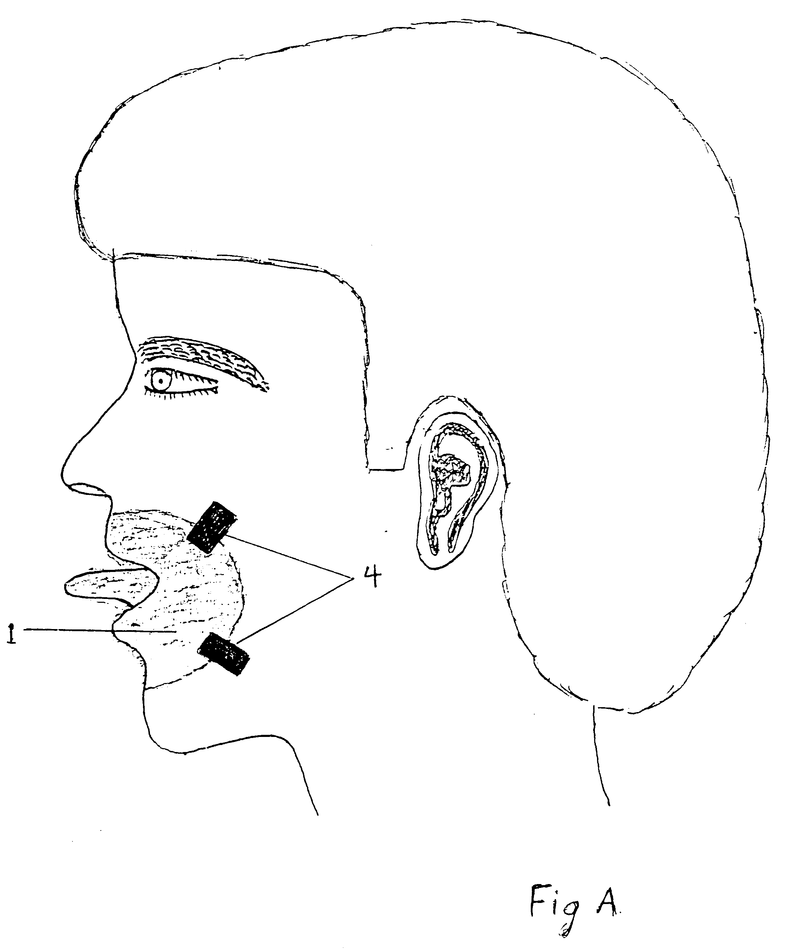

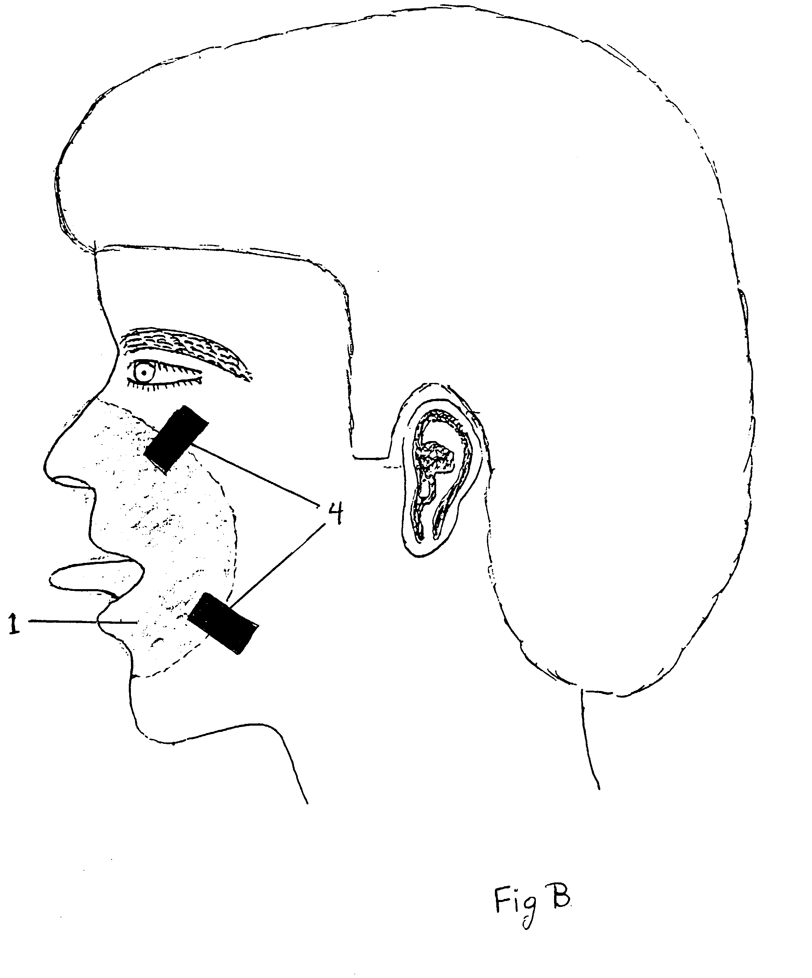

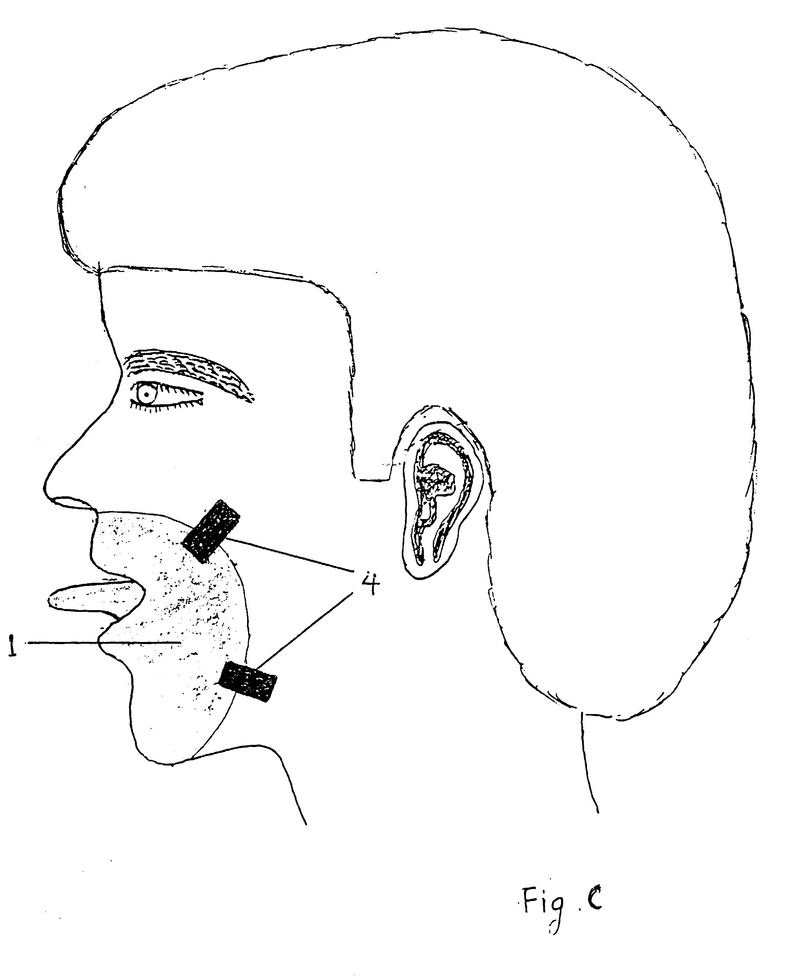

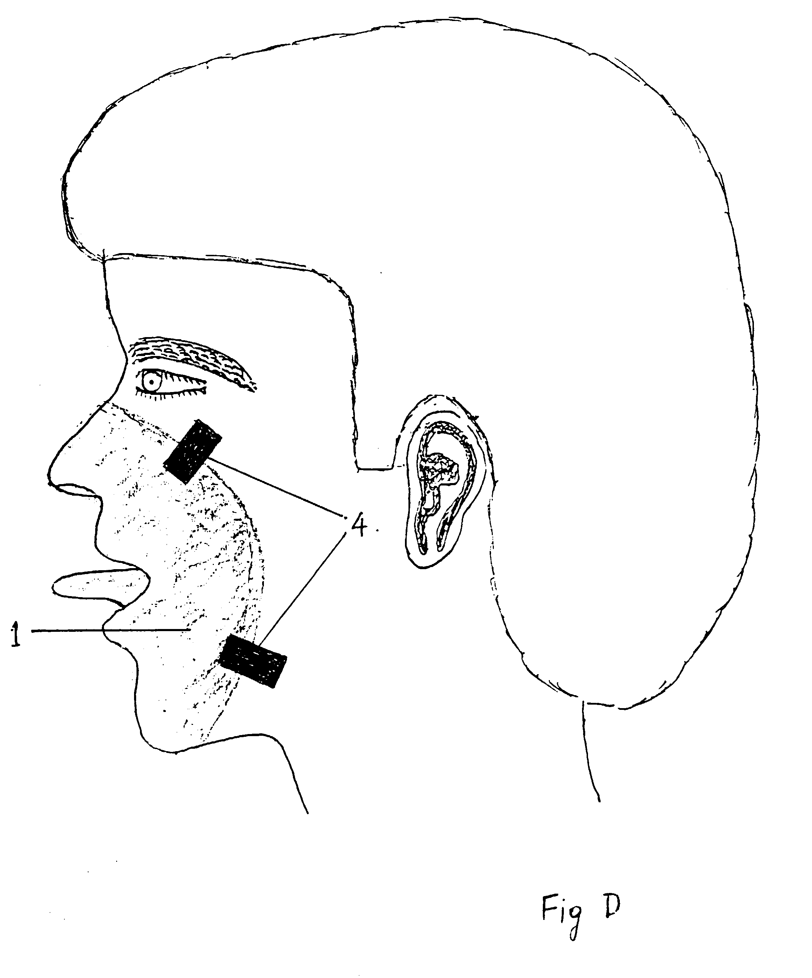

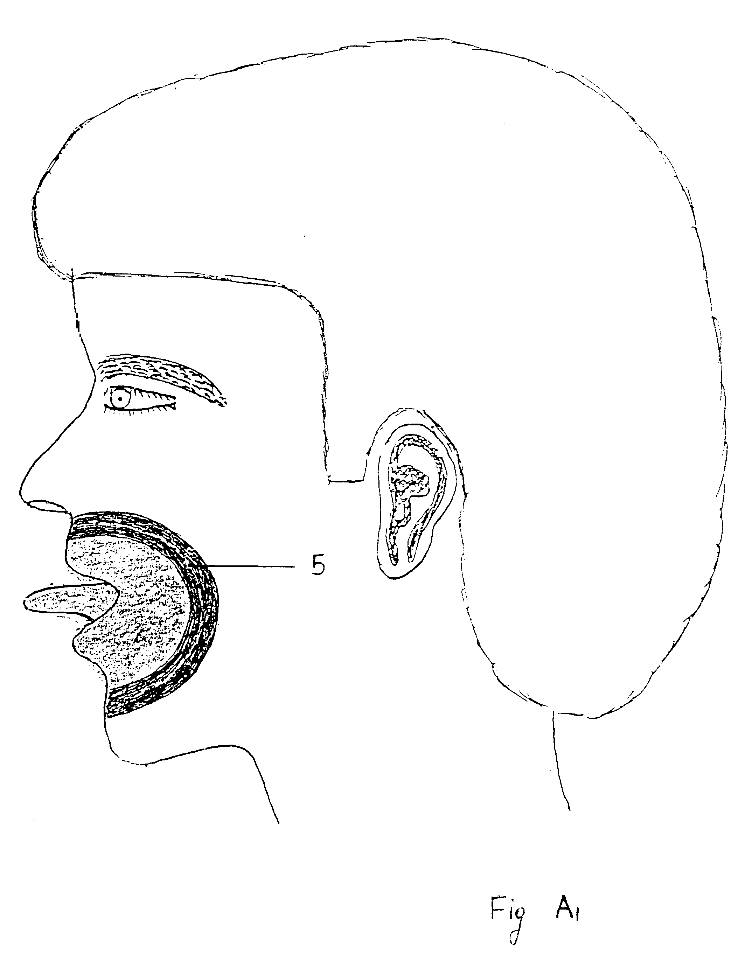

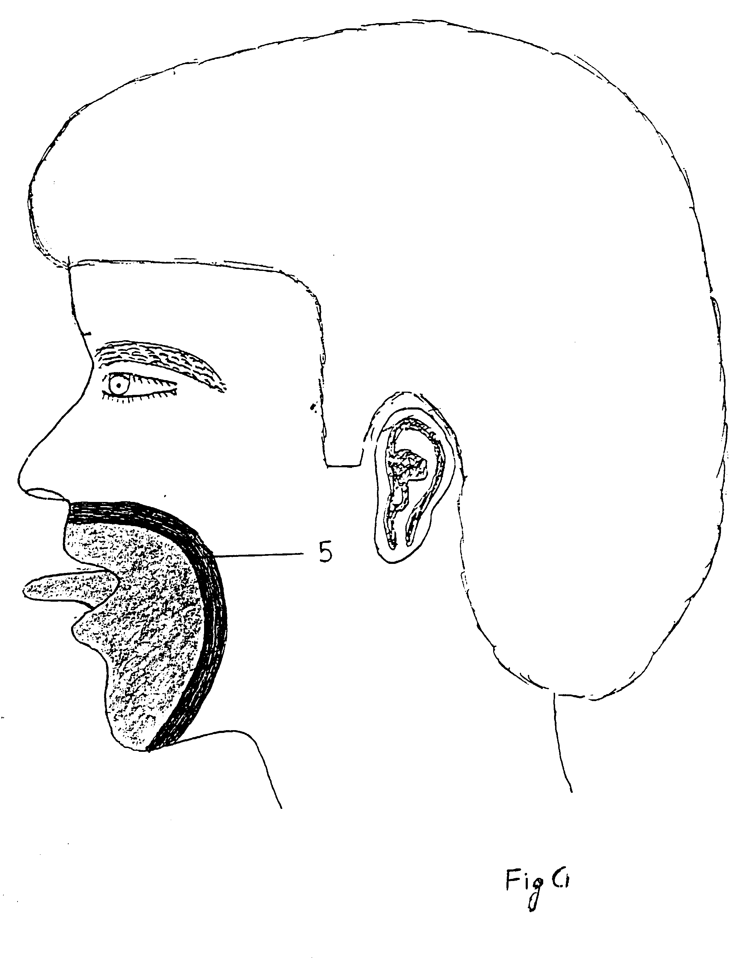

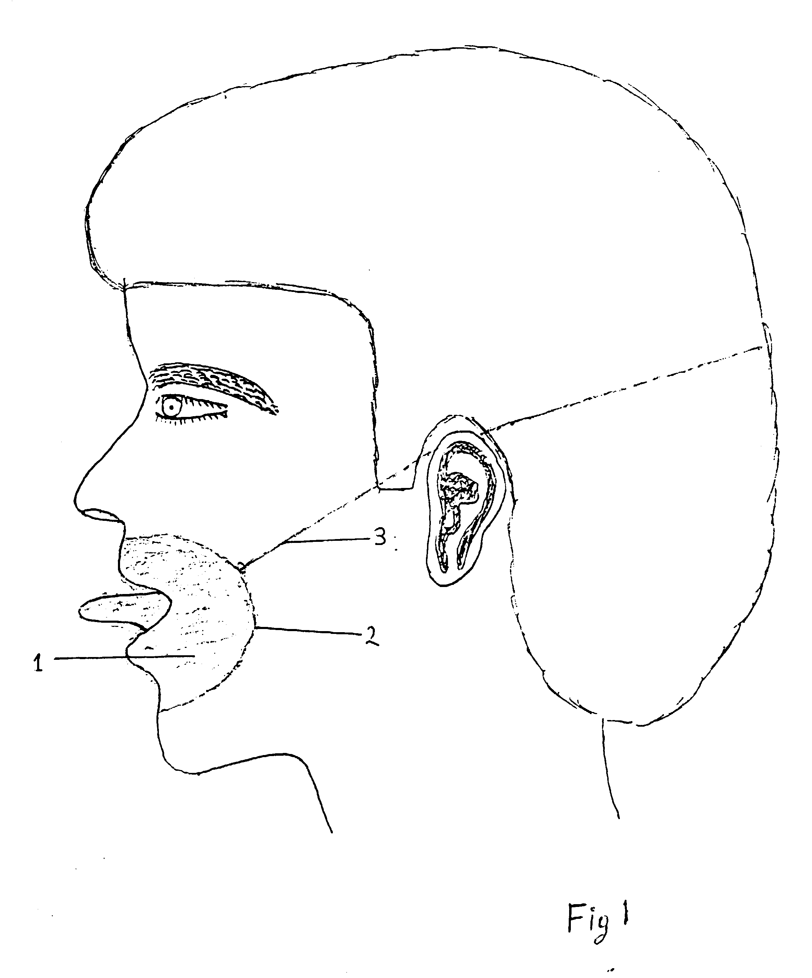

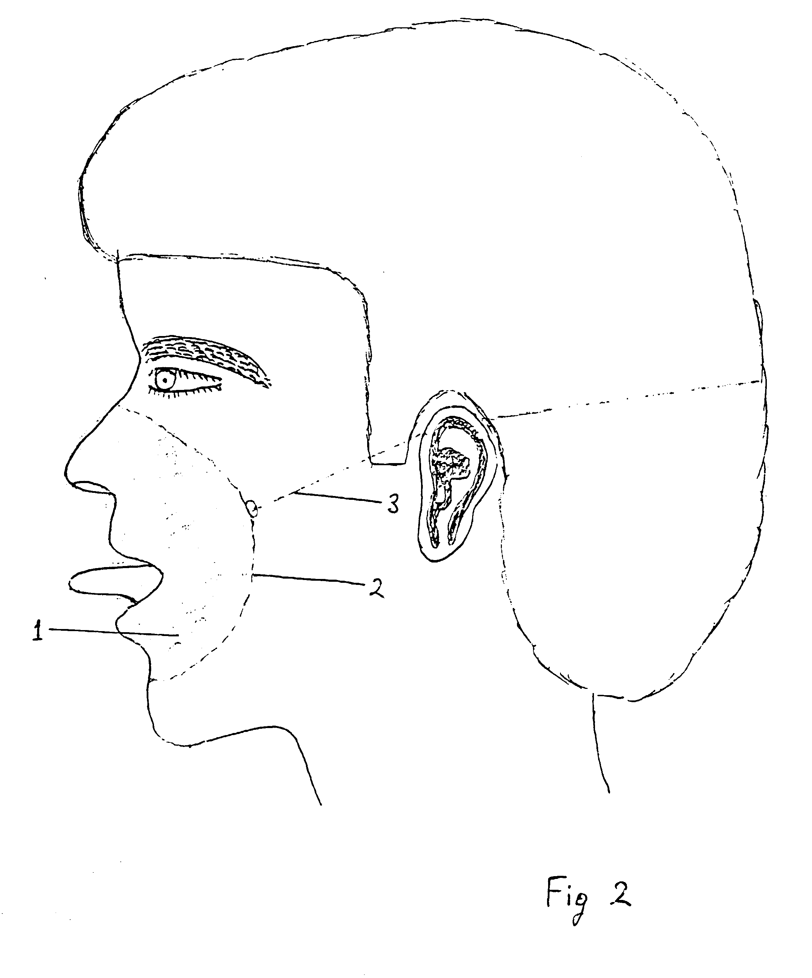

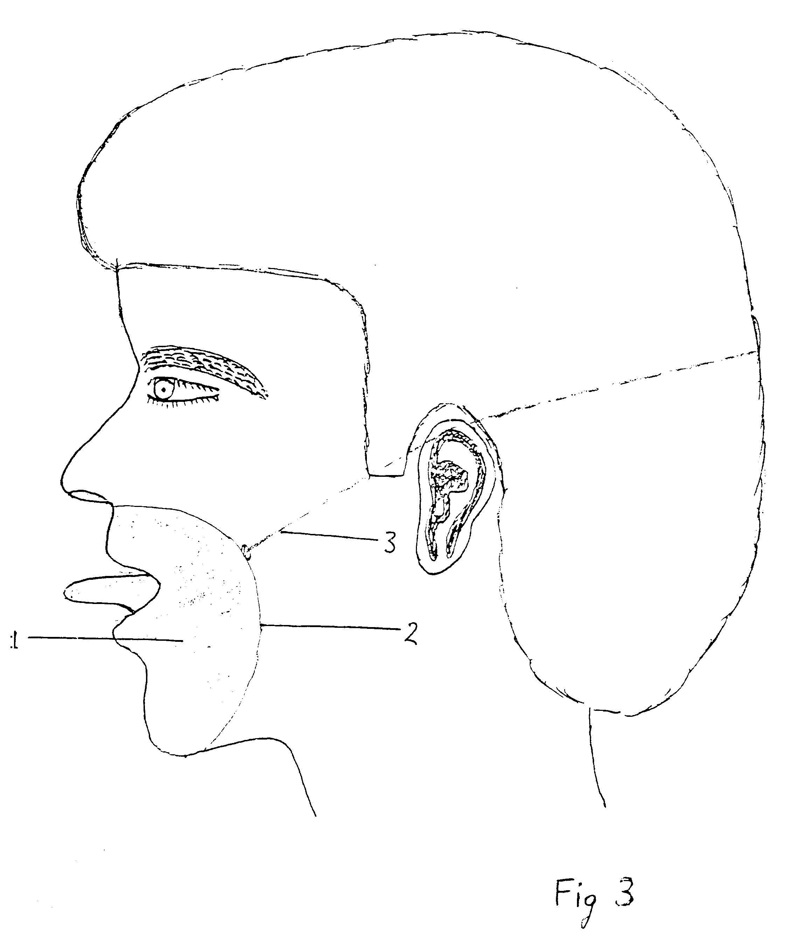

This invention concerns a protective sheath to be used during oral sex. It consists of an elastic sheath (1) which is surrounded by a thin plastic "wire" frame (2)to hold it in position.. At one point on each side - right and left, at the level of the cheeks this wire frame will have an attachment point for an elastic cord (3) which fits round the head (Fig. 1, 2, 3, 4). The sheath can also be held with self-adhesive ribbons (4) applied to specially reinforced points of the sheath , at the other end on the person's cheeks (Fig. A, B, C, D).

The fine plastic "wire" (2) will fit around the mouth and the lips. At the top, it will cover the skin to the nasal-labial groove and at the lower ring will reach the labial-chin groove. This is the smallest and most convenient version (Fix. 11).

A frame of similar thin plastic wire (2) like the one in Fig. 1, will cover the area from the root of the nose to the chin-lips groove (Fig. 21).

A frame (2) of the same material of the previous Figures 1,2, will cover the area upwards to the nose-lip line and downwards to under the chin (fig. 3).

A frame of similar thin plastic wire as that of Fig. 1, 2 and 3, will be used in this case too.

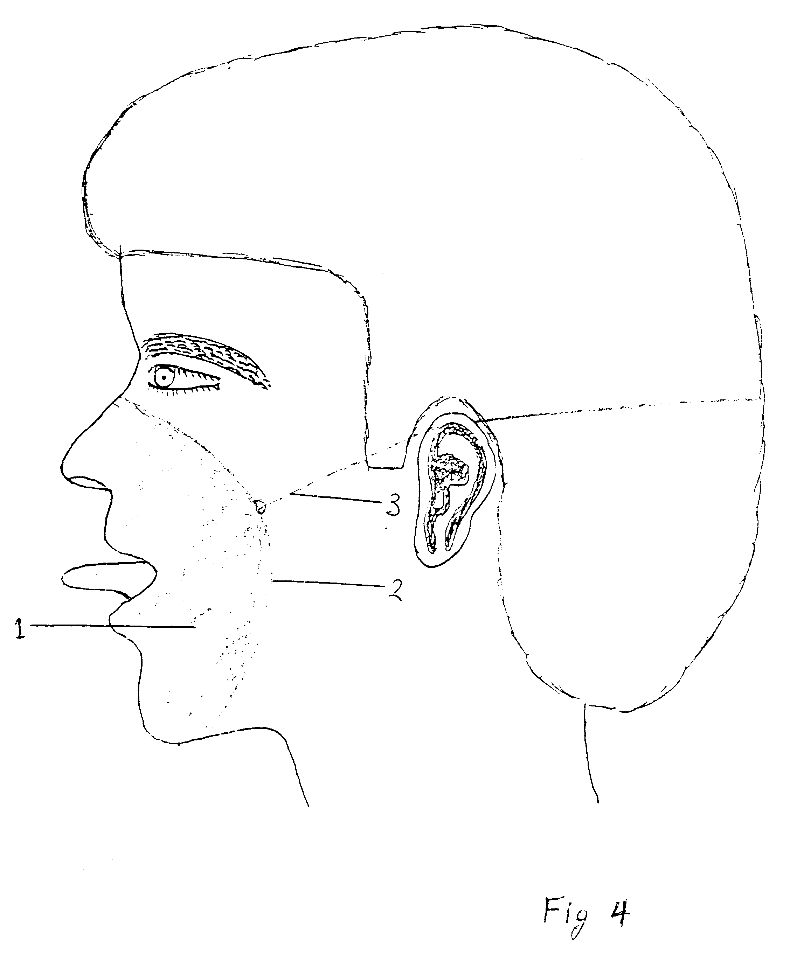

It will however be larger than in the other arrangements for greater safety as it will cover up to the root of the nose and down to below the chin (Fig. 4).

I will mention here that the elastic sheath (1) which in the cases as above is retained by a plastic frame (2) and the elastic cord round the head (3) can in this case be held in place with self-adhesive ribbons (4) (Fig. A, B, C, D) which, as mentioned above, will attach to the sheath rim and to the cheeks.

In the cases of the Illustrations A and C, the sheath can also be secured by a ribbon all round the sheath's periphery, in addition to th e self-adhesive ribbons (Fig. A1, Fig. C1).

The value of this invention is self-evident, as it provides protection of both love-makers from the passage from the one to the other of AIDS, Hepatitis and Germs.

## Claims

1. The sheath for oral sex, in this invention, cosists of an elastic sheath (1) which receives the protruding tongue; a frame (2) that holds the sheath, a round-the- head cord (3) holding the frame (2). This frame can vary in size according to the extent of the area around the mouth that is required to protect (Fig. 1, 2, 3, 4).

2. The protective sheath for oral sex is in this case similar to the elastic sheath (1) mentioned under 1, but the sheath is held by self-adjesive ribbons (4) (Fig. A, B, C, D) instead of the frame and the elastic cord, These ribbons (4) are at one end fixed onto specially reinforced points of the sheath's perihery; at the other end, the ribbons are affixed on to the wearer's cheeks.

3. The elastic sheath (1) may also be attached by self-adhesive tape (5) on the periphery of the sheath (Fig. A1, C1).
